(19) Europäisches Patentamt
European Patent Office
Office européen des brevets

(11) Veröffentlichungsnummer: **0 133 282 B1**

(12) # EUROPÄISCHE PATENTSCHRIFT

(45) Veröffentlichungstag der Patentschrift: **23.10.91**

(21) Anmeldenummer: **84108766.1**

(22) Anmeldetag: **25.07.84**

(51) Int. Cl.5: **C12N 15/31**, C12P 21/00, C07K 1/00, C07K 15/04, C12P 21/02, C07K 7/08, C07K 7/10

(54) **Herstellung von Polypeptiden mit einem Säureamid-Carboxyterminus.**

(30) Priorität: **27.07.83 DE 3327007**

(43) Veröffentlichungstag der Anmeldung:
**20.02.85 Patentblatt 85/08**

(45) Bekanntmachung des Hinweises auf die Patenterteilung:
**23.10.91 Patentblatt 91/43**

(84) Benannte Vertragsstaaten:
**AT BE CH DE FR GB IT LI LU NL SE**

(56) Entgegenhaltungen:
**EP-A- 0 108 387**

**Science, vol. 218 n 4572, 5 November 1982 (Washington DC). R. Guillemin et al "Growth hormone-releasing factor from a human pancreatic tumor that caused acromegaly", pages 585-587**

(73) Patentinhaber: **HOECHST AKTIENGESELLSCHAFT**
**Postfach 80 03 20**
**W-6230 Frankfurt am Main 80(DE)**

(72) Erfinder: **Engels, Joachim, Dr.**
**Feldbergstrasse 1**
**W-6242 Kronberg/Taunus(DE)**
Erfinder: **König, Wolfgang, Dr.**
**Eppsteiner Strasse 25**
**W-6238 Hofheim am Taunus(DE)**
Erfinder: **Müllner, Hubert, Dr.**
**Pestalozzistrasse 4**
**W-6233 Kelkheim (Taunus)(DE)**
Erfinder: **Uhlmann, Eugen, Dr.**
**Fuchstanzstrasse 16**
**W-6240 Königstein/Taunus(DE)**
Erfinder: **Wetekam, Waldemar, Dr.**
**Zeilring 40/I**
**W-6239 Eppstein/Taunus(DE)**

**EP 0 133 282 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Nature, vol. 298, n 5874, 12 August 1982 (New York, London). A.F. Bradbury et al "Mechanism of C-terminal amide formation by pituitary enzymes", pages 686-688

Febs Letters, vol. 152, n 2, 21 February 1983 (Amsterdam). I. Husain et al "Formation of the COOH-terminal amide group of thyrotropin-releasing-factor", pages 277-281

## Beschreibung

Die Erfindung betrifft ein Verfahren zur Herstellung von Polypeptiden mit einem Carbonsäureamid-Carboxyterminus, neue Peptide aus dieser Reihe und dafür geeignete Genstrukturen, Plasmide und Wirtsorganismen.

Die direkte gentechnologische Synthese von Polypeptiden mit einem Carbonsäureamid-Carboxyterminus ist nicht möglich. Es wurden jedoch eine ganze Reihe von natürlichen Peptiden mit einem Carboxamid-Terminus isoliert, wie PHI, VIP, CRF, Nerve Growth Factor, Oxytocin, Cholecystokinin, Gastrin, Calcitonin, Vasopressin, Mellitin, Melanotropin und insbesondere der Wachstumshormon-Releasing Factor, im folgenden "GRF" genannt.

In der EP-A 0 070 675 wird diskutiert, daß man Human-Calcitonin dadurch herstellen könnte, daß man zunächst ein Vorprodukt herstellt, bei dem am C-terminalen Ende ein weiteres Glycin translatiert wird. Dieses Glycin soll dann enzymatisch in die Carbonamidgruppe der vorhergehenden Aminosäure umgewandelt werden. Diese Umwandlung soll mit Hefe-Carboxypeptidase Y vorgenommen werden, wozu jedoch dieses Enzym nicht geeignet ist.

Die Erfindung betrifft insbesondere die Herstellung von Peptiden mit der Aminosäuresequenz von GRF und dem GRF ähnlichen, modifizierten Peptiden und Teilsequenzen dieser Peptide. Im folgenden wird deshalb zunächst das Wesen der Erfindung an der Herstellung von Peptiden mit der Aminosäuresequenz von GRF und von modifizierten GRF-Derivaten erläutert:

GRF wird im Hypothalamus gebildet und stimuliert in der Hypophyse die Sekretion des Wachstumshormons. Bisher konnte GRF jedoch nicht aus menschlichen Hypothalamus-Extrakten isoliert und charakterisiert werden. Aus einem humanen Pankreas-Tumor konnte jedoch ein Peptid aus 44 Aminosäuren isoliert werden, das in vitro und in vivo GRF-Aktivität zeigt (R. Guillemin et al., Science 218 (1982) 585-587). Dieses Peptid hat die folgende Aminosäure-Sequenz:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-Arg-Gly-Ala-Arg-Ala-
Arg-Leu-$NH_2$.

Der genetische Code ist bekanntlich "entartet", d.h. daß nur für zwei Aminosäuren eine einzige Nucleotid-Sequenz codiert, während den restlichen 18 genetisch codierbaren Aminosäuren 2 bis 6 Tripletts zuzuordnen sind. Für die Synthese des Gens besteht somit eine unübersehbare Vielfalt von Codon-Möglichkeiten. Es wurde nun gefunden, daß die DNA-Sequenz I besonders vorteilhaft ist.

Am 5'-Ende des codierenden Stranges befindet sich eine "überhängende" DNA-Sequenz, entsprechend der Restriktionsendonuclease EcoRI, am 3'-Ende des codierenden Strangs dagegen die einzelsträngige, überhängende Sequenz entsprechend dem Restriktionsenzym SalI. Diese beiden unterschiedlichen Erkennungssequenzen gewährleisten die Insertion der DNA in Plasmide in der gewünschten Orientierung.

Zwischen diesen Erkennungssequenzen und den Codons für die Aminosäuren-Folge befindet sich am 5'-Ende des codierenden Stranges das Codon für die Aminosäure Methionin (das in der DNA-Sequenz I mit 0 beziffert ist). Am Ende dieses Stranges folgt auf das für Leucin codierende Triplett das Codon 45 für Glycin und 2 Terminations-Tripletts.

Eine interne singuläre Schnittstelle für das Restriktionsenzym PstI (im Codon 24 des codierenden Strangs bzw. Codon 23 des nicht codierenden Strangs) ermöglicht die Subklonierung zweier Genfragmente, die in gut untersuchte Klonierungsvektoren, wie etwa pBR 322 oder pUC 8, eingebaut werden können. Zusätzlich wurden innerhalb des Strukturgens eine Reihe von weiteren singulären Erkennungssequenzen für Restriktionsendonucleasen eingebaut, die einerseits einen Zugang zu Teilsequenzen des GRF schaffen und andererseits die Durchführung von Mutationen erlauben:

| Restriktionsenzym | Schnitt nach Nukleotid Nr. (cod. Strang) | Peptidfragment Aminosäure | |
|---|---|---|---|
| | | von | bis |
| Pvu II | 56 | 1 | 16 |
| Pst I | 79 | 1 | 24 |
| Xba I | 90 | 1 | 29 |
| Hinf I | 105 | 1 | 34 |
| Bst N I | 114 | 1 | 36 |
| Xho I | 127 | 1 | 41 |

Die DNA-Sequenz I läßt sich aus 13 Oligonukleotiden mit einer Länge von 18 bis 29 Nukleotiden aufbauen, indem diese zunächst chemisch synthetisiert und dann über "sticky ends" von 4 bis 6 Nukleotiden enzymatisch verknüpft werden.

Bei der DNA-Sequenz I wurde weiterhin berücksichtigt, daß bei denjenigen Aminosäuren, denen mehrere Codons zuzuordnen sind, diese nicht gleichwertig sind, sondern vielmehr in der jeweiligen Wirtszelle wie E. coli unterschiedliche Präferenzen zeigen. Weiterhin wurden palindromische Sequenzen auf ein Mindestmaß reduziert.

Die Genstruktur der DNA-Sequenz I ist somit leicht aus relativ kleinen Bausteinen zugänglich, ermöglicht die Subklonierung zweier Genfragmente in gut bekannte Vektoren und erlaubt deren Expression in hoher Ausbeute. Je nach dem Einbau das synthetischen Gens in den Klonierungsvektor wird das erwünschte Peptid mit der Aminosäuresequenz des GRF unmittelbar oder in Form eines Fusionsproteins mit einem bakteriellen Protein wie $\beta$-Galactosidase exprimiert. Solche Fusionsproteine werden dann in an sich bekannter Weise chemisch oder enzymatisch gespalten.

Als Beispiel für eine Teilsequenz, die zu einem Peptid mit den ersten 16 Aminosäuren des GRF mit terminaler Carbonamidgruppe (und einem Methionin-Rest am Aminoende) führt, steht eine abgewandelte DNA-Sequenz I, bei der an das Triplett Nr. 16 unmittelbar die Tripletts Nr. 45-47 anschließen. Entsprechendes gilt für die Peptidfragmente, die in der vorstehenden Tabelle aufgeführt sind und die Aminosäuren bis zur Nr. 24, 29, 34, 36 und 41 aufweisen. Analog kann auch beispielsweise eine Teilsequenz mit den ersten 26 Aminosäuren hergestellt werden.

Als Beispiel für einen modifizierten GRF sei ein Polypeptid genannt, bei dem in Position 27 das Methionin durch Leucin ersetzt ist. Hierzu wird in der DNA-Sequenz I das Triplett Nr. 27 durch das folgende ersetzt:

```
27
Leu
CTG
GAC
```

Weitere Variationen ergeben sich aus der DNA-Sequenz II, von der - zur Vereinfachung - nur der codierende Strang dargestellt ist.

Erfindungsgemäß kann somit ein Polypeptid der Formel I

$$Y - R - NH_2 \quad (I)$$

in der Y den Methioninrest oder einen über Methionin gebundenen Rest eines Bakterienproteins und R eine Peptidsequenz aus genetisch codierbaren Aninosäuren bedeutet, dadurch hergestellt werden, daß man gentechnologisch ein Polypeptid der Formel II

$$Y - R - NH - CH_2 - COOH \quad (II)$$

erzeugt und das Produkt enzymatisch in das Polypeptid der Formel I umwandelt.

Wenn R die Aminosäuresequenz des GRF bedeutet, wobei in Position 27 Leu steht, so kann das Methionin am Aminoende durch Bromcyan-Spaltung abgetrennt werden. Wird bei der gentechnologischen Synthese ein Fusions-Protein erhalten, so wird der unerwünschte Anteil des Bakterienproteins bei der

Bromcyan-Spaltung mit entfernt.

Die enzymatische Umwandlung des Glycylrestes in die Aminogruppe der Carbonamidfunktion ist an sich bekannt (A.F. Bradbury et al., Nature 298 (1982) 686-688; I.Husain et al., FEBS Letters 152 (1983) 277-281).

Der Einbau des synthetischen Gens bzw. Genfragments in Klonierungsvektoren, beispielsweise die Plasmide pUC 8, pBR 322, pKK 177.3, ptac 11 und andere handelsübliche bzw. allgemein zugängliche Plasmide erfolgt in an sich bekannter Weise. Hierzu kann auf das Lehrbuch von Maniatis (Molecular Cloning, Maniatis et al., Cold Spring Harbor, 1982) verwiesen werden.

Die Transformation der so erhaltenen Hybridplasmide in geeignete Wirtsorganismen, vorteilhaft E. coli, ist ebenfalls an sich bekannt und in dem vorstehend genannten Lehrbuch eingehend beschrieben. Die Gewinnung des exprimierten Proteins, die Bromcyan-Spaltung und die Aufarbeitung sind von Guillemin (a.a.O.) beschrieben.

Die erfindungsgemäß erhaltenen Polypeptide mit dem Methioninrest am Aminoende und/oder dem Glycinrest am Carboxy-Ende sind neu und ebenfalls Gegenstand der Erfindung. Dasselbe gilt für die modifizierten GRF-Derivate gemäß DNA-Sequenz II, die DNA-Sequenzen I und II, die genannten Genfragmente, die damit erhaltenen Hybridplasmide, die transformierten Wirtsorganismen und die exprimierten Fusionsproteine.

Weitere Ausgestaltungen der Erfindung sind in den Patentansprüchen niedergelegt.

In den folgenden Beispielen werden noch einige Ausgestaltungen der Erfindung im einzelnen erläutert, woraus sich die Vielzahl der möglichen Abwandlungen und Kombinationen für den Fachmann ergeben. Prozentangaben beziehen sich hierbei auf das Gewicht, wenn nichts anderes angegeben ist.

## Beispiele

### 1. Chemische Synthese eines einzelsträngigen Oligonucleotids

Am Beispiel des Genbausteins Ia, der die Nucleotide 1-27 des codierenden Strangs umfaßt, wird die Synthese der Genbausteine erläutert. Nach bekannten Methoden (M.J. Gait et al., Nucleic Acids Res. 8 (1980) 1081-1096) wird das am 3'-Ende stehende Nucleosid, im vorliegenden Falle also Adenosin (Nucleotid Nr. 27), an Kieselgel ($^{(R)}$FRACTOSIL, Firma Merck) über die 3'-Hydroxyfunktion covalent gebunden. Hierzu wird zunächst das Kieselgel unter Abspaltung von Ethanol mit 3-(Triethoxysilyl)-propylamin umgesetzt, wobei eine Si-O-Si-Bindung entsteht. Das Adenosin wird als $N^6$-Benzoyl-3'-O-succinoyl-5'-dimethoxytritylether in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylamingruppe acyliert.

In den folgenden Syntheseschritten wird die Basenkomponente als 5'-O-Dimethoxytrityl-nucleosid-3'-phosphorigsäure-monomethylester-dialkylamid oder -chlorid eingesetzt, wobei das Adenin als $N^6$-Benzoyl-Verbindung, das Cytosin als $N^4$-Benzoyl-Verbindung, das Guanin als $N^2$-Isobutyryl-Verbindung und das keine Aminogruppe enthaltende Thymin ohne Schutzgruppe vorliegen.

100 mg des polymeren Trägers, der 4 µmol Adenin gebunden enthält, werden nacheinander mit den folgenden Agentien behandelt:

a) Nitromethan
b) gesättigte Zinkbromidlösung in Nitromethan mit 1 % Wasser
c) Methanol
d) Tetrahydrofuran
e) Acetonitril
f) 80 µmol des entsprechenden Nucleosidphosphits und 400 µmol Tetrazol in 1 ml wasserfreiem Acetonitril (5 Minuten)
g) 20 % Acetanhydrid in Tetrahydrofuran mit 40 % Lutidin und 10 % Dimethylaminopyridin (2 Minuten)
h) Tetrahydrofuran
i) Tetrahydrofuran mit 20 % Wasser und 40 % Lutidin
j) 3 % Jod in Kollidin/Wasser/Tetrahydrofuran im Volumenverhältnis 5:4:1
k) Tetrahydrofuran und
l) Methanol.

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion (b) spektrophotometrisch durch Messung der Absorption des Dimethoxytritylkations bei einer Wellenlänge von 496 nm bestimmt werden.

Nach abgeschlossener Synthese des Oligonucleotids werden die Methylphosphatschutzgruppen des Oligomers mit Hilfe von p-Thiokresol und Triethylamin abgespalten. Anschließend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Genbausteine Ib - IIg synthetisiert, deren Nucleotidfolge aus der DNA-Sequenz III hervorgeht.

2. Enzymatische Verknüpfung der einzelsträngigen Oligonucleotide zu den Genfragmenten I und II

Zur Phosphorylierung der Oligonucleotide am 5'-Terminus wird je 1 nmol der Oligonucleotide Ia und Ib mit 5 nmol Adenosintriphosphat mit vier Einheiten T4-Polynucleotid-Kinase in 20 µl 50 mM Tris-HCl-Puffer (pH 7,6), 10 mM Magnesiumchlorid und 10 mM Dithiothreitol (DTT) 30 Minuten bei 37°C behandelt (C.C. Richardson, Progress in Nucl. Acids Res. 2 (1972) 825). Das Enzym wird durch fünfminütiges Erhitzen auf 95°C desaktiviert. Anschließend werden die Oligonucleotide Ia und Ib gegeneinander hybridisiert, indem man sie in wäßriger Lösung 2 Minuten auf 95°C erhitzt und dann langsam auf 5°C abkühlt.

Analog werden die Oligonucleotide Ic und Id sowie Ie und If phosphoryliert und paarweise hybridisiert.

Für das Genfragment II werden die Oligonucleotide IIa mit IIb sowie IIf mit IIg paarweise hybridisiert und die verbleibenden Oligonucleotide IIc, IId und IIe gemeinsam hybridisiert.

Die so erhaltenen drei Oligo-Nucleotidpaare für das Genfragment I bzw. die beiden Oligo-Nucleotidpaare und der Oligo-Nucleotidtripel für das Genfragment II werden wie folgt ligiert:
Die doppelsträngigen Nucleotide werden vereinigt und in jeweils 40 µl 50 mM Tris-HCl-Puffer, 20 mM Magnesiumchlorid und 10 mM DTT mit Hilfe von 100 Einheiten T4-DNA-Ligase bei 15°C im Lauf von 16 Stunden ligiert.

Die Reinigung der Genfragmente I und II erfolgt durch Gelelektrophorese auf einem 15%igen Polyacrylamidgel (ohne Harnstoffzusatz, 20 x 40 cm, 1 mm Dicke), wobei als Markersubstanz ØX 174 DNA (Fa. BRL), geschnitten mit Hinf I, diente.

3. Herstellung von Hybridplasmiden, die die Genfragmente I und II enthalten

a) Einbau des Genfragments I in pBR 322

Das handelsübliche Plasmid pBR 322 wird in bekannter Weise mit den Restriktionsendonucleasen EcoRI und PstI nach den Angaben der Hersteller geöffnet. Der Verdauungsansatz wird auf einem 5%igen Polyacrylamidgel durch Elektrophorese in bekannter Weise aufgetrennt und die Bruchstücke durch Anfärben mit Ethidiumbromid oder durch radioaktive Markierung ("Nick-Translation" nach Maniatis, a.a.O. ) sichtbar gemacht. Die Plasmidbande wird anschließend aus dem Acrylamidgel ausgeschnitten und elektrophoretisch vom Polyacrylamid abgetrennt.

1 µg Plasmid wird dann mit 10 ng Genfragment I über Nacht bei 16°C ligiert. Man erhält das Hybridplasmid gemäß Figur 1.

b) Einbau des Genfragments II in pUC 8

Analog zu a) wird das handelsübliche Plasmid pUC 8 mit PstI und SalI aufgeschnitten und mit dem Genfragment II ligiert. Man erhält das Hybridplasmid gemäß Figur 2.

c) Einbau eines modifizierten Genfragments

Entsprechend b) kann auch ein modifiziertes Genfragment II eingebaut werden, bei dem - entsprechend der DNA-Sequenz II im Nucleotid-Triplett Nr. 27 das Codon für Leucin eingesetzt ist. Dieses modifizierte Genfragment II wird durch eine entsprechend abgewandelte Synthese der Genbausteine IIa und IIb erhalten, wobei im Baustein IIa die Sequenz ATG durch CTG und entsprechend dem Genbaustein IIb die Sequenz TAC durch GAC ersetzt werden.

4. Synthese des kompletten Gens

a) Transformation und Amplification

Die so erhaltenen Hybridplasmide werden in E. coli transformiert. Hierzu wird der Stamm E. coli K 12 durch Behandlung mit einer 70 mM Calciumchloridlösung kompetent gemacht und mit der Suspension des Hybridplasmids in 10 mM Tris-HCl-Puffer (pH 7,5) oder 70 mM Calciumchloridlösung versetzt. Die transformierten Stämme werden wie üblich unter Ausnutzung der durch das Plasmid vermittelten Antibiotikaresistenzen bzw. -empfindlichkeiten selektioniert und die Hybridvektoren amplifiziert. Nach Abtöten der Zellen werden die Hybridplasmide isoliert, mit den ursprünglich eingesetzten Restriktionsenzymen aufgeschnitten und die Genfragmente I und II durch Gelelektrophorese isoliert.

b) Verknüpfung der Genfragmente

Die durch Amplifikation erhaltenen Genfragmente Ia und Ib werden wie im Beispiel 2 beschrieben enzymatisch verknüpft und das so erhaltene synthetische Gen mit der DNA-Sequenz I in Klonierungsvektoren eingeführt.

5. Synthese von Hybridplasmiden, die das komplette Gen enthalten

a) Einbau in pKK 177.3

Das Expressionsplasmid pKK 177.3 (Plasmid ptac 11, Amman et al., Gene 1983, 167-178, bei dem in die EcoRI-Erkennungsstelle synthetisch eine Sequenz eingebaut wurde, die eine SalI-Schnittstelle enthält) wird mit den Restriktionsenzymen EcoRI und SalI geöffnet und mit dem synthetischen Gen entsprechend der DNA-Sequenz I ligiert. Hierbei wird das eingeführte Gen hinter die Regulationsregion des Plasmids pKK 177.3 gesetzt. Nach Zugabe eines geeigneten Inductors wie Isopropyl-β-thiogalacto-pyranosid (IPTG) wird eine mRNA gebildet, die zur Expression des Polypeptids Met-GRF-Gly führt.

b) Einbau in pWH1

Das Expressionsplasmid pWH 1 besteht aus einem pBR 322-Anteil und dem Gen für β-Galactosidase mit dessen Regulationsregion (Figur 3). Zu dessen Herstellung werden 10 µg pBR 322 mit den Restriktionsendonucleasen EcoRI und PvuII geschnitten und in 5%igem Polyacrylamidgel elektrophoretisch aufgetrennt. Nach Sichtbarmachung der DNS-Banden mit Ethidiumbromid (oder durch radioaktive Markierung) wird die größere Bande (2292 Basenpaare) aus dem Gel herausgeschnitten und durch Elektroelution vom Polyacrylamid befreit. Aus einem Lac-transduzierenden Phagen wie λ oder Ø 80 dlac wird das Gen für β-Galactosidase durch Verdauung mit EcoRI und Partialverdauung mit PvuII herausgespalten. Analog zu der vorstehend beschriebenen Aufarbeitung wird ein Fragment von ungefähr 3185 Nucleotidpaaren isoliert. Dieses Fragment trägt die intakte Regulationsregion des Lac-Operons und die genetische Information der β-Galactosidase mit den Aminosäuren 1 bis 1005. Durch Ligieren dieses Lac-DNA-Fragments in das DNA-Fragment aus dem Plasmid pBR 322 resultiert das Plasmid pWH 1 mit der Regulationsregion vom Lac-Operon, der Codierungssequenz der β-Galactosidase (Aminosäuren 1 bis 1005), dem Ampicillin-Resistenzgen von pBR 322 und der Replikationsregion von pBR 322. Hierbei kann die natürliche Regulationsregion des Lac-Operons durch beliebige synthetische Regulationsregionen, wie beispielsweise tac, ersetzt werden. Dieses Plasmid enthält bei der Aminosäure 1005 der β-Galactosidase eine singuläre EcoRI-Schnittstelle, die als Klonierungsstelle für eukaryotische Gene benutzt werden kann.

An die DNA-Sequenz I wird ein chemisch-synthetischer Linker anligiert, der die Erkennungssequenzen für die Restriktionsenzyme SalI und EcoRI enthält:

```
5'-TCGACGCCCG
            GCGGGCTTAA - 5'
```

Nach einer Restriktionsenzymverdauung mit EcoRI erhält man ein DNA-Fragment, flankiert von EcoRI-Erkennungssequenzen. Ein solches Fragment kann anschließend in das mit EcoRI geöffnete Plasmid pWH1 integriert werden.

Hierzu wird pWH1 mit EcoRI geöffnet und mit bakterieller alkalischer Phosphatase an den 5'-Enden der

EcoRI-Schnittstelle dephosphoryliert. Hierdurch wird eine Ligierung des Plasmids mit sich selber verhindert und erheblich der "Hintergrund" transformierter Bakterienkulturen mit nicht rekombinantem Plasmid erniedrigt. Nur diejenigen Plasmide können ringförmig geschlossen werden, welche ein Genfragment mit flankierenden EcoRI-Erkennungssequenzen integriert haben.

6. Transformation der Hybridplasmide

Kompetente E. coli-Zellen werden mit 0,1 - 1 μg Plasmid pWH1 transformiert, welches mit 10 μg GRF-Gen zusammenligiert wurde, und auf Ampicillin-Agarplatten plattiert. Anschließend läßt sich die Integration des GRF-Gens und seine Integrationsrichtung im Plasmid durch DNA-Schnellaufarbeitung bestimmen (Maniatis, a.a.O.).

7. Expression

Nach Transformation dieser Hybridplasmide in E. coli wird ein Fusionsprotein exprimiert, das am Aminoende der Met-GRF-Sequenz die 1005 Aminosäuren der β-Galactosidase trägt. Nach Bromcyan-Spaltung wird ein GRF-Gly-Derivat erhalten.

Analog hierzu werden modifizierte GRF-Proteine erhalten, beispielsweise das Produkt, bei dem als Aminosäure Nr. 27 Leucin anstelle von Methionin steht.

8. Aufarbeitung und Reinigung

Die zur gewünschten optischen Dichte kultivierten Bakterienstämme werden mit einem geeigneten Induktor, beispielsweise IPTG, hinreichend lange, beispielsweise 2 Stunden, induziert. Anschließend werden die Zellen mit 0,1 % Kresol und 0,1 mM Phenyl-methyl-sulfonylfluorid (Benzylsulfonylfluorid) abgetötet. Nach Zentrifugieren oder Filtrieren wird die Zellmasse in einer wäßrig-sauren Lösung bei pH 3,0 in einer geeigneten Vorrichtung (French-Presse bzw. (R)Dyno-Mühle) aufgeschlossen, worauf die unlöslichen Bestandteile abzentrifugiert werden. Aus dem Überstand werden die Proteine nach der Methode von Guillemin (a.a.O.) isoliert. Durch HPLC-Analytik werden die Anreicherung und die Reinheit der Produkte kontrolliert.

Das Methionin (bzw. bei Fusionsproteinen einschließlich des Anteils des am Methionin gebundenen Bakterienproteins wie β-Galactosidase) wird durch Bromcyan-Spaltung abgetrennt und das GRF-Derivat sauer extrahiert und gereinigt (Guillemin, a.a.O.).

Fusionsproteine mit einem β-Galactosidase-Anteil lassen sich bereits im Rohextrakt der lysierten Bakterien anhand ihres unterschiedlichen Laufverhaltens von der authentischen β-Galactosidase in der Gelelektrophorese nachweisen. Nach der Bromcyanspaltung werden - wie vorstehend erwähnt - Reinheitsgrad und Anreicherung des GRF-Derivats und seiner Varianten über HPLC bestimmt.

Die Charakterisierung der Produkte auf GRF-Aktivität kann immunologisch und biologisch erfolgen:
In einem Radioimmunoassay reagiert das bakteriologisch hergestellte GRF und seine Varianten mit einem Antikörper gegen synthetisches GRF mit der Aminosäuresequenz 1-44. Der Radioimmunoassay wird zweckmäßig als indirekter Immunpräzipitationsassay mit Anti-GRF-Antikörper und Anti-IgG-Anti-GRF-Antikörper ausgestaltet. Die Empfindlichkeit des RIA beträgt 10 ng GRF.

Beim biologischen Test werden das bakteriologisch hergestellte Produkt und seine Varianten in Ratten auf Wachstumshormonfreisetzung getestet. Hierzu wird anaesthesierten Ratten das Produkt oder seine Varianten intravenös appliziert und die Freisetzung des Wachstumshormons im Blut der Ratte radioimmunologisch nachgewiesen.

DNA-Sequenz I

| | | | | | | | |
|---|---|---|---|---|---|---|---|
| Triplett-Nr. | | | | 0 | 1 | 2 | 3 |
| Aminosäure | | | | Met | Tyr | Ala | Asp |
| Nucleotid-Nr. | | 1 | 5 | | 10 | | 15 |
| Codierender Strang | 5' | AA | TTC | ATG | TAC | GCT | GAC |
| nicht codierender Strang | 3' | | G | TAC | ATG | CGA | CTG |

| | | | | | | | | | |
|---|---|---|---|---|---|---|---|---|---|
| 4 | 5 | 6 | 7 | 8 | 9 | 10 | 11 | 12 | 13 |
| Ala | Ile | Phe | Thr | Asn | Ser | Tyr | Arg | Lys | Val |
| 20 | | 25 | | 30 | 35 | | 40 | | 45 |
| GCT | ATC | TTC | ACT | AAC | TCT | TAC | CGT | AAA | GTT |
| CGA | TAG | AAG | TGA | TTG | AGA | ATG | GCA | TTT | CAA |

| | | | | | | | | | |
|---|---|---|---|---|---|---|---|---|---|
| 14 | 15 | 16 | 17 | 18 | 19 | 20 | 21 | 22 | 23 |
| Leu | Gly | Gln | Leu | Ser | Ala | Arg | Lys | Leu | Leu |
| 50 | | 55 | | 60 | 65 | | 70 | | 75 |
| CTG | GGT | CAG | CTG | TCT | GCT | CGT | AAA | CTG | CTG |
| GAC | CCA | GTC | GAC | AGA | CGA | GCA | TTT | GAC | GAC |

| | | | | | | | | | |
|---|---|---|---|---|---|---|---|---|---|
| 24 | 25 | 26 | 27 | 28 | 29 | 30 | 31 | 32 | 33 |
| Gln | Asp | Ile | Met | Ser | Arg | Gln | Gln | Gly | Glu |
| 80 | | 85 | | 90 | 95 | | 100 | | 105 |
| CAG | GAC | ATC | ATG | TCT | AGA | CAG | CAG | GGT | GAA |
| GTC | CTG | TAG | TAC | AGA | TCT | GTC | GTC | CCA | CTT |

| | | | | | | | | | |
|---|---|---|---|---|---|---|---|---|---|
| 34 | 35 | 36 | 37 | 38 | 39 | 40 | 41 | 42 | 43 |
| Ser | Asn | Gln | Glu | Arg | Gly | Ala | Arg | Ala | Arg |
| 110 | | 115 | | 120 | 125 | | 130 | | 135 |
| TCT | AAC | CAG | GAA | CGT | GGT | GCT | CGA | GCT | CGT |
| AGA | TTG | GTC | CTT | GCA | CCA | CGA | GCT | CGA | GCA |

| | | | | | | |
|---|---|---|---|---|---|---|
| 44 | 45 | 46 | 47 | | | |
| Leu | Gly | Stp | Stp | | | |
| 140 | | 145 | 149 | | | |
| CTG | GGT | TAA | TAG | | | 3' |
| GAC | CCA | ATT | ATC | AGC | T | 5' |

DNA-Sequenz II (codierender Strang)

| | | | | | | | | | | | | | |
|---|---|---|---|---|---|---|---|---|---|---|---|---|---|
| 0 | 1 | 2 | 3 | 4 | 5 | 6 | 7 | 8 | 9 | 10 | 11 | 12 | 13 |
| ATG | 1A1 | GCT | GAC | GCT | ATC | TTC | ACT | A2C | TCT | TAC | CGT | A2A | 2TT |
| Met | Tyr | Ala | Asp | Ala | Ile | Phe | Thr | Asn | Ser | Tyr | Arg | Lys | Val |
| | His | | | | | | | Ser | | | | Arg | Ile |
| 14 | 15 | 16 | 17 | 18 | 19 | 20 | 21 | 22 | 23 | 24 | 25 | 26 | 27 |
| CTG | GGT | CAG | CTG | T31 | GCT | CGT | AAA | CTG | CTG | CA4 | GA3 | ATC | 3TG |
| Leu | Gly | Gln | Leu | Ser | Ala | Arg | Lys | Leu | Leu | Gln | Asp | Ile | Met |
| | | | | Tyr | | | | | | His | Glu | | Leu |
| 28 | 29 | 30 | 31 | 32 | 33 | 34 | 35 | 36 | 37 | 38 | 39 | 40 | 41 |
| abc | AGA | CAG | CAG | GGT | GAA | deT | AAC | CAG | GAA | Clm | 5GT | GCT | CGA |
| Ser | Arg | Gln | Gln | Gly | Glu | Ser | Asn | Gln | Glu | Arg | Gly | Ala | Arg |
| Asn | | | | | | Arg | | | | Gln | Arg | | |
| 42 | 43 | 44 | 45 | 46 | 47 | | | | | | | | |
| fg1 | hi1 | CTG | GGT | TAA | TAG | | | | | | | | |
| Ala | Arg | Leu | Gly | Stp | Stp | | | | | | | | |
| Phe | Asn | | | | | | | | | | | | |

1 = T oder C
2 = A oder G
3 = C oder A
4 = G oder T
5 = G oder C

abc = TCT oder AAC
de = TC oder CG
fg = GC oder TT
hi = AA oder CG
lm = AA, AG, GA, GG oder GT

DNA-Sequenz III

```
Triplett-Nr.                              0     1     2     3
                              Ia
Codierender Strang       5'  AA    TTC   ATG   TAC   GCT   GAC
nicht codierender Strang 3'        G     TAC   ATG   CGA   CTG
                              Ib

 4     5     6     7     8     9     10    11    12    13
       Ia                Ic
GCT   ATC   TTC   ACT   AAC   TCT   TAC   CGT   AAA   GTT
CGA   TAG   AAG   TGA   TTG   AGA   ATG   GCA   TTT   CAA
       Ib                      Id
 14    15    16    17    18    19    20    21    22    23

       Ic                Ie
CTG   GGT   CAG   CTG   TCT   GCT   CGT   AAA   CTG   CTG
GAC   CCA   GTC   GAC   AGA   CGA   GCA   TTT   GAC   GAC
       Id                If                        IIb
 24    25    26    27    28    29    30    31    32    33

Ie                IIa                        IIc
CAG   GAC   ATC   ATG   TCT   AGA   CAG   CAG   GGT   GAA
GTC   CTG   TAG   TAC   AGA   TCT   GTC   GTC   CCA   CTT
                  IIb                           IId
 34    35    36    37    38    39    40    41    42    43

             IIc                           IIf
TCT   AAC   CAG   GAA   CGT   GGT   GCT   CGA   GCT   CGT
AGA   TTG   GTC   CTT   GCA   CCA   CGA   GCT   CGA   GCA
       IId               IIe                          Ig
 44    45    46    47

       IIf
CTG   GGT   TAA   TAG               3'
GAC   CCA   ATT   ATC   AGC   T     5'
       IIg
```

## Patentansprüche

## Patentansprüche für folgende Vertragsstaaten: BE, CH, DE, FR, GB, IT, LI, LU, NL, SE

1. Verfahren zur Herstellung eines Polypeptids der Formel I

$$Y - R - NH_2 \quad (I)$$

in der Y den Methioninrest oder einen über Methionin gebundenen Rest eines Bakterienproteins und R eine Peptidsequenz aus genetisch codierbaren Aminosäuren bedeutet, dadurch gekennzeichnet, daß man gentechnologisch ein Polypeptid der Formel II

$$Y - R - NH - CH_2 - COOH \quad (II)$$

erzeugt und das Produkt enzymatisch in das Polypeptid der Formel I umwandelt.

2. Verfahren nach Anspruch 1, dadurch gekennzeichnet, daß man gentechnologisch ein Peptid der Formel II erzeugt, in dem R keinen Methioninrest enthält, und anschließend den Rest Y durch Bromcyan-Spaltung abtrennt.

3. Verfahren nach Anspruch 1 oder 2, dadurch gekennzeichnet, daß R die Aminosäuresequenz (GRF 1 bis n) bedeutet, in der GRF für Wachstumshormon-Releasing Factor oder eine Modifikation dieser Sequenz steht und n 16 bis 44 bedeutet.

4. Verfahren nach einem oder mehreren der Ansprüche 1 bis 3, dadurch gekennzeichnet, daß bei der gentechnologischen Erzeugung des Polypeptids der Formel II hinter dem für Glycin codierenden Triplett 2 Stop-Codons vorgesehen sind.

5. Polypeptid der Formel IIa

$$Met - R - NH - CH_2 - COOH \quad (II^{a)}$$

in der R die in Anspruch 3 genannte Bedeutung hat.

6. Polypeptid der Formel IV

$$H - R - Gly \quad (IV)$$

in der R die Aminosäuresequenz des GRF enthält, wobei jedoch in Position 27 Leu statt Met steht.

7. Fusionsproteine der Formel II, in der R die im Anspruch 3 genannte Bedeutung hat und wobei an die Aminogruppe des Methioninrests ein bakterielles Protein gebunden ist.

8. DNA-Sequenz der Formel V

```
5' - AA TTC ATG -              -CTG CA
                   (GRF 1-22)           (V)
3' -      G TAC -              -G.
```

in der (GRF 1-22) die Codons für die Aminosäuren 1-22 von GRF und seinen Modifikationen bedeutet.

9. DNA-Sequenz der Formel VI

```
          G  -                        - GGT TAA TAG
3'-AC GTC  -  (GRF 25-44)             - CCA ATT ATC AGC T
```

in der (GRF 25-44) die Codons für die Aminosäuren 25-44 von GRF und seinen Modifikationen bedeutet.

**10.** DNA-Sequenz der Formel VII

```
5' - AA TTC ATG -                 -GGT TAA TAG           (VII)
3' -      G TAC -  (GRF 1 bis n)  -CCA ATT ATC AGC T
```

in der (GRF 1 bis n) die Codons für die Aminosäuren 1 bis n des GRF und seinen Modifikationen bedeutet, wobei n für 16 bis 44 steht.

**11.** Hybridplasmide, die zwischen einer EcoRI- und einer PstI-Schnittstelle eine DNA-Sequenz der Formel V enthalten.

**12.** Hybridplasmide, die zwischen einer PstI- und einer SalI-Schnittstelle eine DNA-Sequenz der Formel VI enthalten.

**13.** Hybridplasmide, die zwischen einer EcoRI- und SalI-Schnittstelle eine DNA-Sequenz der Formel VII enthalten.

**14.** Wirtsorganismen, die Hybridplasmide nach Anspruch 11-13 enthalten.

**Patentansprüche für folgenden Vertragsstaat: AT**

**1.** Verfahren zur Herstellung eines Polypeptids der Formel I

$$Y - R - NH_2 \quad (I)$$

in der Y den Methioninrest oder einen über Methionin gebundenen Rest eines Bakterienproteins und R eine Peptidsequenz aus genetisch codierbaren Aminosäuren bedeutet, dadurch gekennzeichnet, daß man gentechnologisch ein Polypeptid der Formel II

$$Y - R - NH - CH_2 - COOH \quad (II)$$

erzeugt und das Produkt enzymatisch in das Polypeptid der Formel I umwandelt.

**2.** Verfahren nach Anspruch 1, dadurch gekennzeichnet, daß man gentechnologisch ein Peptid der Formel II erzeugt, in dem R keinen Methioninrest enthält, und anschließend den Rest Y durch Bromcyan-Spaltung abtrennt.

**3.** Verfahren nach Anspruch 1 oder 2, dadurch gekennzeichnet, daß R die Aminosäuresequenz (GRF 1 bis n) bedeutet, in der GRF für Wachtumshormon-Releasing Factor oder eine Modifikation dieser Sequenz steht und n 16 bis 44 bedeutet.

**4.** Verfahren nach einem oder mehreren der Ansprüche 1 bis 3, dadurch gekennzeichnet, daß bei der gentechnologischen Erzeugung des Polypeptids der Formel II hinter dem für Glycin codierenden Triplett 2 Stop-Codons vorgesehen sind.

**5.** Verfahren nach einem oder mehreren der Ansprüche 1 bis 4, dadurch gekennzeichnet, daß man zur

gentechnologischen Erzeugung des Polypeptids der Formel II eine DNA-Sequenz der Formel V

```
5' - AA TTC ATG -                  -CTG CA
                     (GRF 1-22)                  (V)
3' -        G TAC -                -G
```

in der (GRF 1-22) die Codons für die Aminosäuren 1-22 von GRF und seinen Modifikationen bedeutet, synthetisiert, diese DNA-Sequenz zwischen eine EcoRI- und eine PstI-Schnittstelle eines Plasmids einbaut, sowie eine DNA-Sequenz der Formel VI

```
          G -                   - GGT TAA TAG
               (GRF 25-44)
3'-AC GTC -                     - CCA ATT ATC AGC T
```

in der (GRF 25-44) die Codons für die Aminosäuren 25-44 von GRF und seinen Modifikationen bedeutet, synthetisiert, diese DNA-Sequenz zwischen eine PstI- und eine SalI-Schnittstelle eines Plasmids einbaut, die erhaltenen Hybridplasmide in eine Wirtszelle transformiert, dort amplifiziert, die DNA-Sequenzen der Formel V und VI reisoliert, ligiert, die ligierte DNA-Sequenz zwischen eine EcoRI- und eine SalI-Schnittstelle eines Plasmids einbaut, das so erhaltene Hybridplasmid in eine Wirtszelle transformiert und zur Expression bringt.

6. Verfahren nach einem oder mehreren der Ansprüche 1 bis 4, dadurch gekennzeichnet, daß man zur gentechnologischen Erzeugung des Polypeptids der Formel II eine DNA-Sequenz der Formel VII

```
5' - AA TTC ATG-                 -GGT TAA TAG
                  (GRF 1 bis n)                       (VII)
3' -        G TAC-               -CCA ATT ATC AGC T
```

in der (GRF 1 bis n) die Codons für die Aminosäuren 1 bis n des GRF und seiner Modifikationen bedeutet, wobei n für 16 bis 44 steht, synthetisiert, zwischen eine EcoRI- und eine SalI-Schnittstelle eines Plasmids einbaut, das so erhaltene Hybridplasmid in eine Wirtszelle transformiert und zur Expression bringt.

**Claims**

**Claims for the following Contracting States: BE, CH, DE, FR, GB, IT, LI, LU, NL, SE**

1. A process for the preparation of a polypeptide of the formula I

$Y - R - NH_2$ (I)

in which Y denotes the methionine residue or a residue of a bacterial protein bonded via methionine, and R denotes a peptide sequence of genetically codable amino acids, which comprises producing a polypeptide of the formula II

$Y - R - NH - CH_2 - COOH$ (II)

by genetic engineering methods, and converting the product enzymatically into the polypeptide of the formula I.

2. The process as claimed in claim 1, wherein a peptide of the formula II, in which R does not contain a methionine residue, is produced by genetic engineering methods, and then the residue Y is removed by cleavage with cyanogen bromide.

3. The process as claimed in claim 1 or 2, wherein R denotes the amino acid sequence (GRF 1 to n), in which GRF represents growth hormone releasing factor or a modification of this sequence, and n

denotes 16 to 44.

4. The process as claimed in one or more of claims 1 to 3, wherein 2 stop codons are provided behind the triplet coding for glycine in the production of the polypeptide of the formula II by genetic engineering methods.

5. A polypeptide of the formula IIa

Met - R - NH - $CH_2$ - COOH (IIa)

in which R has the meaning mentioned in claim 3.

6. A polypeptide of the formula IV

H - R - Gly (IV)

in which R contains the amino acid sequence of GRF, but with Leu in place of Met in position 27.

7. Fused proteins of the formula II in which R has the meaning mentioned in claim 3, and where a bacterial protein is bonded to the amino group of the methionine residue.

8. A DNA sequence of the formula V

```
5' - AA TTC ATG -              -CTG CA
                  (GRF 1-22)                (V)
3' -        G TAC -            -G
```

in which (GRF 1-22) denotes the codons for the amino acids 1-22 of GRF and its modifications.

9. A DNA sequence of the formula VI

```
          G -                 - GGT TAA TAG
                 (GRF 25-44)
3'-AC GTC -                   - CCA ATT ATC AGC T
```

in which (GRF 25-44) denotes the codons for the amino acids 25-44 of GRF and its modifications.

10. A DNA sequence of the formula VII

```
5' - AA TTC ATG -                  -GGT TAA TAG   (VII)
                   (GRF 1 to n)
3' -        G TAC -                -CCA ATT ATC AGC T
```

in which (GRF 1 to n) denotes the codons for the amino acids 1 to n of GRF and its modifications, where n represents 16 to 44.

11. Hybrid plasmids which contain a DNA sequence of the formula V between an EcoRI and a PstI cleavage site.

12. Hybrid plasmids which contain a DNA sequence of the formula VI between a PstI and a SalI cleavage site.

13. Hybrid plasmids which contain a DNA sequence of the formula VII between an EcoRI and a SalI cleavage site.

**14.** Host organisms which contain hybrid plasmids as claimed in claims 11-13.

**Claims for the following Contracting State: AT**

**1.** A process for the preparation of a polypeptide of the formula I

$$Y - R - NH_2 \quad (I)$$

in which Y denotes the methionine residue or a residue of a bacterial protein bonded via methionine, and R denotes a peptide sequence of genetically codable amino acids, which comprises producing a polypeptide of the formula II

$$Y - R - NH - CH_2 - COOH \quad (II)$$

by genetic engineering methods, and converting the product enzymatically into the polypeptide of the formula I.

**2.** The process as claimed in claim 1, wherein a peptide of the formula II, in which R does not contain a methionine residue, is produced by genetic engineering methods, and then the residue Y is removed by cleavage with cyanogen bromide.

**3.** The process as claimed in claim 1 or 2, wherein R denotes the amino acid sequence (GRF 1 to n), in which GRF represents growth hormone releasing factor or a modification of this sequence, and n denotes 16 to 44.

**4.** The process as claimed in one or more of claims 1 to 3, wherein 2 stop codons are provided behind the triplet coding for glycine in the production of the polypeptide of the formula II by genetic engineering methods.

**5.** The process as claimed in one or more of claims 1 to 4, wherein for the production of the polypeptide of the formula II by genetic engineering methods a DNA sequence of the formula V

```
5' - AA TTC ATG -              -CTG CA
                  (GRF 1-22)                (V)
3' -       G TAC -              -G
```

in which (GRF 1-22) denotes the codons for the amino acids 1-22 of GRF and its modifications is synthesized, this DNA sequence is incorporated between an EcoRI and a PstI cleavage site of a plasmid, and a DNA sequence of the formula VI

```
          G -                - GGT TAA TAG
                (GRF 25-44)
   3'-AC GTC -               - CCA ATT ATC AGC T
```

in which (GRF 25-44) denotes the codons for the amino acids 25-44 of GRF and its modifications is synthesized, this DNA sequence is incorporated between a PstI and a SalI cleavage site of a plasmid, the resulting hybrid plasmids are transformed into a host cell and amplified therein, the DNA sequences of the formula V and VI are reisolated and ligated, the ligated DNA sequence is incorporated between an EcoRI and a SalI cleavage site of a plasmid, and the hybrid plasmid obtained in this way is transformed into a host cell and is expressed.

**6.** The process as claimed in one or more of claims 1 to 4, wherein for the production of the polypeptide of the formula II by genetic engineering methods a DNA sequence of the formula VII

```
5' - AA TTC ATG -                  -GGT TAA TAG
                      (GRF 1 to n)                    (VII)
3' -        G TAC-                 -CCA ATT ATC AGC T
```

in which (GRF 1 to n) denotes the codons for the amino acids 1 to n of GRF and its modifications, where n represents 16 to 44, is synthesized and incorporated between an EcoRI and a SalI cleavage site of a plasmid, and the hybrid plasmid obtained in this way is transformed into a host cell and is expressed.

**Revendications**

**Revendications pour les Etats conractants suivants: BE, CH, DE, FR, GB, IT, LI, LU, NL, SE**

**1.** Procédé pour la production d'un polypeptide de formule I

$Y\text{-}R\text{-}NH_2$ (I)

dans laquelle Y représente le reste méthionine ou un reste d'une protéine bactérienne liée par la méthionine, et R représente une séquence peptidique d'aminoacides génétiquement codables, caractérisé en ce que l'on produit par génie génétique un polypeptide de formule II

$Y\text{-}R\text{-}NH\text{-}CH_2\text{-}COOH$ (II)

et on convertit par voie enzymatique le produit en le polypeptide de formule I.

**2.** Procédé selon la revendication 1, caractérisé en ce que l'on produit par génie génétique un peptide de formule II, dans lequel R ne contient pas de reste méthionine, et on sépare ensuite le reste Y par coupure au bromure de cyanogène.

**3.** Procédé selon la revendication 1 ou 2, caractérisé en ce que R représente la séquence d'aminoacides (GRF 1 à n) dans laquelle GRF représente le facteur déclenchant la sécrétion de l'hormone somatotrope, ou une modification de cette séquence, et n va de 16 à 44.

**4.** Procédé selon une ou plusieurs des revendications 1 à 3, caractérisé en ce que, dans la production par génie génétique du polypeptide de formule II, deux codons d'arrêt sont prévus derrière le triplet codant pour la glycine.

**5.** Polypeptide de formule IIa

$Met\text{-}R\text{-}NH\text{-}CH_2\text{-}COOH$ (IIa)

dans laquelle R a la signification donnée dans la revendication 3.

**6.** Polypeptide de formule IV

H-R-Gly (IV)

dans laquelle R contient la séquence d'aminoacides du GRF, Leu étant toutefois à la place de Met en position 27.

**7.** Protéine fusionnée de formule II, dans laquelle R a la signification donnée dans la revendication 3 et dans laquelle le reste méthionine d'une protéine bactérienne est lié au groupe amino.

**8.** Séquence d'ADN de formule V

```
5' - AA TTC ATG -              - CTG CA
                  (GRF 1-22)                  (V)
3' -        G TAC -            - G
```

dans laquelle (GRF 1-22) représente les codons codant pour les aminoacides 1-22 du GRF et de ses modifications.

**9.** Séquence d'ADN de formule VI

```
          G -               - GGT TAA TAG
              (GRF 22-44)                       (VI)
3'-AC GTC -                 - CCA ATT ATC AGC T
```

dans laquelle (GRF 25-44) représente les codons codant pour les aminoacides 25-44 du GRF et de ses modifications.

**10.** Séquence d'ADN de formule VII

```
5' - AA TTC ATG -              - GGT TAA TAG
                  (GRF 1 à n)                      (VII)
3' -        G TAC -            - CCA ATT ATC AGC T
```

dans laquelle (GRF 1 à n) représente les codons codant pour les aminoacides 1 à n du GRF et de ses modifications, n allant de 16 à 44.

**11.** Plasmides hybrides qui contiennent une séquence d'ADN de formule V entre un site de coupure EcoRI et un site de coupure PstI.

**12.** Plasmides hybrides qui contiennent une séquence d'ADN de formule VI entre un site de coupure PstI et un site de coupure SalI.

**13.** Plasmides hybrides qui contiennent une séquence d'ADN de formule VII entre un site de coupure EcoRI et un site de coupure SalI.

**14.** Organismes hôtes qui contiennent des plasmides hybrides selon les revendications 11 à 13.

**Revendications pour l'Etat contractant suivant: AT**

**1.** Procédé pour la production d'un polypeptide de formule I

$Y\text{-}R\text{-}NH_2$ (I)

dans laquelle Y représente le reste méthionine ou un reste d'une protéine bactérienne liée par la méthionine, et R représente une séquence peptidique d'aminoacides génétiquement codables, caractérisé en ce que l'on produit par génie génétique un polypeptide de formule II

$Y\text{-}R\text{-}NH\text{-}CH_2\text{-}COOH$ (II)

et on convertit par voie enzymatique le produit en le polypeptide de formule I.

**2.** Procédé selon la revendication 1, caractérisé en ce que l'on produit par génie génétique un peptide de formule II, dans lequel R ne contient pas de reste méthionine, et on sépare ensuite le reste Y par coupure au bromure de cyanogène.

**3.** Procédé selon la revendication 1 ou 2, caractérisé en ce que R représente la séquence d'aminoacides (GRF 1 à n) dans laquelle GRF représente le facteur déclenchant la sécrétion de l'hormone somatotrope, ou une modification de cette séquence, et n va de 16 à 44.

**4.** Procédé selon une ou plusieurs des revendications 1 à 3, caractérisé en ce que dans la production par génie génétique du polypeptide de formule II, deux codons d'arrêt sont prévus derrière le triplet codant pour la glycine.

**5.** Procédé selon une ou plusieurs des revendications 1 à 4, caractérisé en ce que, pour la production par génie génétique du polypeptide de formule II, on synthétise une séquence d'ADN de formule V

```
5' - AA TTC ATG -            - CTG CA
                  (GRF 1-22)                (V)
3' -       G TAC -            - G
```

dans laquelle (GRF 1-22) représente les codons codant pour les aminoacides 1-22 du GRF et de ses modifications, on incorpore cette séquence d'ADN entre un site EcoRI et un site PstI d'un plasmide, ainsi qu'une séquence d'ADN de formule VI

```
           G -              - GGT TAA TAG
               (GRF 22-44)                         (VI)
3'-AC GTC -                 - CCA ATT ATC AGC T
```

dans laquelle (GRF 25-44) représente les codons codant pour les aminoacides 25-44 du GRF et de ses modifications, on incorpore cette séquence d'ADN entre un site PstI et un site SalI d'un plasmide, les plasmides hybrides obtenus sont transformés dans une cellule hôte, y sont amplifiés, on isole à nouveau les séquences d'ADN de formules V et VI, on les ligature, on incorpore la séquence d'ADN ligaturée entre un site EcoRI et un site SalI d'un plasmide, le plasmide hybride ainsi obtenu est transformé dans une cellule hôte et exprimé.

**6.** Procédé selon une ou plusieurs des revendications 1 à 4, caractérisé en ce que, pour la production par génie génétique du polypeptide de formule II, on synthétise une séquence d'ADN de formule VII

```
5' - AA TTC ATG -             - GGT TAA TAG
                  (GRF 1 à n)                          (VII)
3' -       G TAC -            - CCA ATT ATC AGC T
```

dans laquelle (GRF 1 à n) représente les codons codant pour les aminoacides 1 à n du GRF et de ses modifications, n allant de 16 à 44, on l'incorpore entre un site EcoRI et un site SalI d'un plasmide, le plasmide hybride ainsi obtenu est transformé dans une cellule hôte et exprimé.

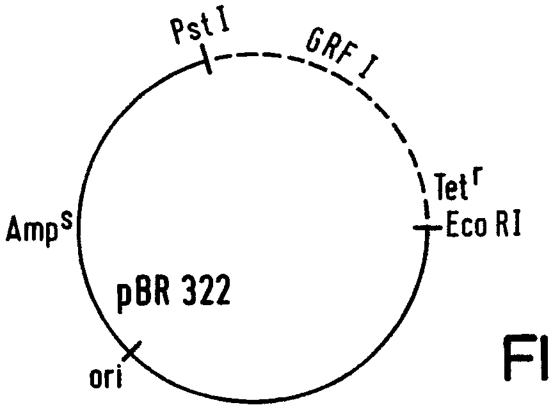


FIG.1

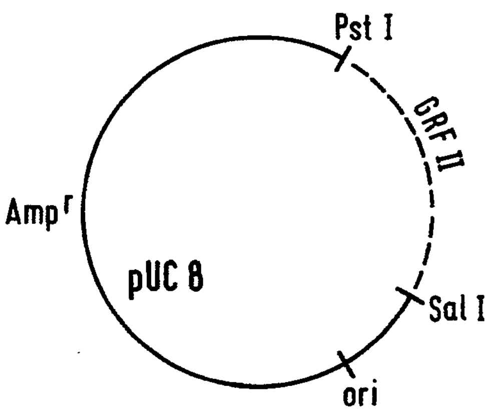


FIG.2

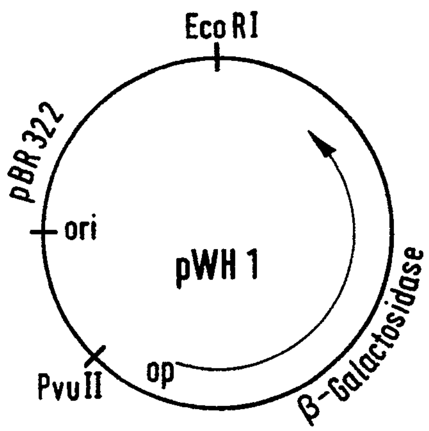


FIG.3